# EUROPEAN PATENT APPLICATION

(11) **EP 4 635 399 A1**
(43) Date of publication of application: **22.10.2025**
(21) Application number: 24745339.2
(22) Date of filing: 24.01.2024
(51) Int. Cl.: A61B 1/04, A61B 1/06, A61B 1/00

(54) **ENDOSCOPE SYSTEM WITH FLUORESCENT MODULE**

(30) Priority: 18.01.2024 CN 202410077005
(71) Applicant: Anhui Dendrite Optical Technology Co., Ltd., Hefei Cty, Anhui Province (CN)
(72) Inventor: CHIANG, Li-yang, Jingan District Shanghai 200040 (CN); WENG, Xianjie, Jingan District Shanghai 200040 (CN)
(74) Representative: Winter, Brandl - Partnerschaft mbB
(86) International application number: PCT/CN2024/073858
(87) International publication number: WO 2025/152201

(57) **Abstract**

An endoscope system having a fluorescence module is provided. An incident light module is fixedly connected to the fluorescence module and provides perpendicular incident light to the fluorescence module. Along a direction perpendicular to the incident light, a rear-end magnifying lens module, the fluorescence module and a lens sheath coaxial module are sequentially arranged from back to front. A relay lens module and an objective lens module are sequentially arranged from back to front inside the lens sheath coaxial module. The fluorescence module includes a cavity body fixedly connected to each of the rear-end magnifying lens module, the incident light module and the lens sheath coaxial module, and a dichroscope is arranged, inside the cavity body, at an angle of 45 degrees with respect to a direction of the incident light.

## Description

### CROSS REFERENCE TO RELATED APPLICATION

This application claims priority to Chinese Patent Application No. 202410077005.8, filed with CNIPA on January 18, 2024, entitled as "ENDOSCOPE SYSTEM HAVING FLUORESCENCE MODULE", the entire content of which is incorporated herein by reference in its entirety.

### TECHNICAL FIELD

The present disclosure relates to the field of medical devices, and in particular, to an endoscope system having a fluorescence module.

### BACKGROUND

An endoscope is a device for examining and treating the internal tissues of the human body by means of a head probe and an optical lens. The optical imaging principle is that light enters the human body from a light source behind the body, is reflected by the inner wall of an endoscope lens, and is ultimately guided by an optical fiber to an observation device. An optical lens of the endoscope focuses the light to form a magnified image for the doctor to observe.

In clinical medicine, in order to achieve fluorescence imaging of the surface of living biological tissue, fluorescence staining will be carried out on the surface of living biological tissue first. However, in addition to excited light rays in a fluorescence wave band, light rays reflected by the surface of living biological tissue may still include a part of reflected light rays of original incident light rays, so optoelectronic conversion at the back-end may be interfered with.

### SUMMARY

In view of the above technical problem, it is necessary to provide an endoscope system having a fluorescence module, which is capable of enabling incident light and emergent light to propagate in an identical tubular cavity while realizing filtration of a specific fluorescence wave band.

An endoscope system having a fluorescence module is provided, the endoscope system includes an incident light module, an objective lens module, a relay lens module, the fluorescence module, a rear-end magnifying lens module, and a lens sheath coaxial module. The incident light module is fixedly connected to the fluorescence module and provides perpendicular incident light to the fluorescence module. Along a direction perpendicular to the incident light, the rear-end magnifying lens module, the fluorescence module and the lens sheath coaxial module are sequentially arranged from back to front; the relay lens module and the objective lens module are sequentially arranged from back to front inside the lens sheath coaxial module. The fluorescence module includes a cavity body fixedly connected to each of the rear-end magnifying lens module, the incident light module and the lens sheath coaxial module, and a dichroscope which is arranged, inside the cavity body, at an angle of 45 degrees with respect to a direction of the incident light. The incident light is totally reflected by the dichroscope, then passes through the relay lens module and reaches the objective lens module, and irradiates a surface of an object under observation, and the incident light is excited by a staining agent on the surface of the object under observation to generate reflected excited fluorescence. The excited fluorescence propagates through the objective lens module and the relay lens module and then reaches a surface of the dichroscope, and is fully transmitted through the dichroscope and enters the rear-end magnifying lens module.

In an embodiment of the present disclosure, the fluorescence module also includes an excitation filter set above the dichroscope and a fluorescence filter set at a back end of the dichroscope, where the incident light strikes vertically onto the excitation filter to generate incident light of 470 nm~480 nm, and the fluorescence filter performs a secondary filtering on excited fluorescence after passing through the dichroscope and generates excited fluorescence of 525 nm±20 nm.

In an embodiment of the present disclosure, the cavity body includes a first connection cavity that opens upwardly for fixedly connecting the incident light module, a second connection cavity that opens backwardly for connecting the rear-end magnifying lens module, and a third connection cavity that opens forwardly for connecting the lens sheath coaxial module, where the first connection cavity, the second connection cavity, and the third connection cavity converge in interior of the cavity body and form an accommodating cavity.

In an embodiment of the present disclosure, the fluorescence module further includes a first fixing member disposed at a front end of the dichroscope and a second fixing member disposed at a back end of the dichroscope, the first fixing member and the second fixing member are affixed to the dichroscope in opposite directions, and the first and second fixing members and the dichroscope are accommodated in the accommodating cavity as a whole.

In an embodiment of the present disclosure, the fluorescence module also includes a fixing washer disposed around a periphery of the dichroscope, the dichroscope is housed in the fixing washer, and the fixing washer and the dichroscope are clamped between the first fixing member and the second fixing member as a whole, forming a fixing module.

In an embodiment of the present disclosure, the fixing module includes a locating face matching an inner wall of the accommodating cavity, the locating face includes a horizontally disposed upper locating face which is rectangular and a horizontally disposed lower locating face having a semi-circular or semi-elliptical cross-section, and the fixing washer includes a horizontally disposed upper support portion which forms a part of the upper locating face, and a horizontally disposed lower support portion which forms a part of the lower locating face.

In an embodiment of the present disclosure, a bottom end of the lower support portion is provided with a first limiting face that fits with a lower inner surface of the cavity body and partially forms the lower locating face, and a second limiting face that perpendicularly intersects with the first limiting face and fits with a front inner surface of the cavity body.

In an embodiment of the present disclosure, a first mounting hole is formed in the first fixing member from top to bottom, and a first cutoff face is formed above the dichroscope; the excitation filter is mounted within the first mounting hole and cuts off at the first cutoff face.

In an embodiment of the present disclosure, a second mounting hole is formed in the second fixing member from back to front, and a second cutoff face is formed at the rear of the dichroscope; the fluorescence filter is mounted within the second mounting hole and cuts off at the second cutoff face.

In an embodiment of the present disclosure, a blocking face is formed at a position where each of the first connection cavity, the second connection cavity and the third connection cavity connects to the cavity body. The incident light module is fixedly connected to the cavity body through the first connection cavity and cuts off at a corresponding blocking face, the rear-end magnifying lens module is fixedly connected to the cavity body through the second connection cavity and cuts off at a corresponding blocking face, and the lens sheath coaxial module is fixedly connected to the cavity body through the third connection cavity and cuts off at a corresponding blocking face.

The above-described endoscope system having a fluorescence module includes an incident light module, an objective lens module, a relay lens module, the fluorescence module, a rear-end magnifying lens module, and a lens sheath coaxial module. The incident light module is fixedly connected to the fluorescence module and provides perpendicular incident light to the fluorescence module. Along a direction perpendicular to the incident light, the rear-end magnifying lens module, the fluorescence module and the lens sheath coaxial module are sequentially arranged from back to front; the relay lens module and the objective lens module are sequentially arranged from back to front inside the lens sheath coaxial module. The fluorescence module includes a cavity body fixedly connected to each of the rear-end magnifying lens module, the incident light module and the lens sheath coaxial module, and a dichroscope which is arranged, inside the cavity body, at an angle of 45 degrees with respect to a direction of the incident light. The incident light is totally reflected by the dichroscope, then passes through the relay lens module and reaches the objective lens module, and irradiates a surface of an object under observation, and the incident light is excited by a staining agent on the surface of the object under observation to generate reflected excited fluorescence. The excited fluorescence propagates through the objective lens module and the relay lens module and then reaches a surface of the dichroscope, and is fully transmitted through the dichroscope and enters the rear-end magnifying lens module. Fluorescence in a specific wave band is filtered by the fluorescence module, which reduces the interference of the optoelectronic conversion at the rear-end magnifying lens, and generates a clearer image, which is convenient for the medical personnel to observe the biological organisms, and to improve the accuracy and efficiency of the medical examination.

### BRIEF DESCRIPTION OF THE DRAWINGS

In order to more clearly illustrate the technical solutions in the embodiments of the present application or in the conventional technology, the accompanying drawings to be used in the description of the embodiments or in the conventional technology will be briefly introduced below, and it will be obvious that the accompanying drawings in the following description are only for the embodiments of the present application, and that for the person of ordinary skill in the field, other attachments can be obtained according to the publicly disclosed accompanying drawings, without putting in creative labor.
FIG. 1 shows a schematic diagram of a cross-section of an incident light module in an endoscope system having a fluorescence module in an embodiment;
FIG. 2 shows a schematic diagram of a cross-section of an objective lens module in an endoscope system having a fluorescence module in an embodiment;
FIG. 3a is a schematic diagram of a section of a front end of a relay lens module in an endoscope system having a fluorescence module in an embodiment;
FIG. 3b is a schematic diagram of a cross-section of a back end of a relay lens module in an endoscope system having a fluorescence module in an embodiment;
FIG. 4 is a schematic diagram of a section of a rear-end magnifying lens module in an endoscope system having a fluorescence module in an embodiment;
FIG. 5 is a schematic diagram of a cross-section of a lens sheath coaxial module in an endoscope system having a fluorescence module in an embodiment;
FIG. 6 is a schematic diagram of a cross-section of a protective lens in an endoscope system having a fluorescence module in an embodiment;
FIG. 7 is a schematic diagram of a cross-section of a fluorescence module in an endoscope system having the fluorescence module in an embodiment;
FIG. 8 shows a schematic diagram of a cross-section of an accommodating cavity in an endoscope system having a fluorescence module in an embodiment;
FIG. 9 is a schematic structural diagram of a self-impacted structure employed in a dichroscope in an endoscope system having a fluorescence module in an embodiment;
FIG. 10 is a schematic diagram of a cross-section of limiting faces of a fixing washer in an endoscope system having a fluorescence module in an embodiment;
FIG. 11 is a schematic diagram of a cross-section of mounting holes in an endoscope system having a fluorescence module in an embodiment;
FIG. 12a is a front view of an endoscope system having a fluorescence module in an embodiment;
FIG. 12b is a right view of an endoscope system having a fluorescence module in an embodiment;
FIG. 12c shows an upper view of an endoscope system having a fluorescence module in an embodiment; and
FIG. 13 shows a schematic diagram of a section in an endoscope system having a fluorescence module in an embodiment.

### DETAILED DESCRIPTION OF THE EMBODIMENTS

An endoscope is a device for examining and treating internal tissues of human body by means of a head probe and an optical lens, whose working principle is based on optical imaging and probe technology, which is used to observe microscopic structures or diseased tissues in the body by introducing a light source and a lens into cavities or tissues of the body. Among other things, the optical imaging principle of the endoscope means that light enters the human body from the light source behind the body, is reflected by an inner wall of an endoscopic lens, and is ultimately guided by an optical fiber to an observation element, and an optical lens of the endoscope focuses the light to form a magnified image for a doctor to observe.

The realization of the optical imaging of the endoscope mainly depends on an optical system provided inside the endoscope. Taking an end where living biological tissue is located as front end, the optical system includes an objective lens, a relay lens set, and an eyepiece lens which are arranged from front end to back end. The objective lens is arranged to collect information of the living biological tissue and generate an image based on the collected information. The relay lens set is arranged to transmit the image. The eyepiece lens is arranged to magnify the image for observation by a clinician. The basis of optical imaging is the information of the living biological tissue collected by the objective lens, so the collection of the information of the living biological tissue is an important and indispensable part.

In clinical medicine, in order to achieve fluorescence imaging of a surface of living biological tissue, fluorescence staining is carried out on the surface of living biological tissue first. However, in addition to excited light rays in a fluorescence wave band, light rays reflected by the surface of living biological tissue may still include a part of reflected light rays of original incident light rays, so optoelectronic conversion at the back-end may be interfered with. In view of this, it is necessary to design a set of overall imaging system in which incident light and emergent light propagate in an identical tubular cavity, and filtration of a specific fluorescence wave band is achieved at the same time.

Technical solutions in embodiments of the present disclosure are described clearly and completely hereinafter in conjunction with accompanying drawings used in the embodiments of the present disclosure. It is obvious that described embodiments are only a part of rather than all of embodiments of the present application. Based on the embodiments of the present disclosure, all other embodiments obtained by a person of ordinary skill in the art without making creative labor all fall within the scope of protection of the present application.

In the description of the present disclosure, it is to be understood that the terms "center", "longitudinal", "lateral", "length ", "width", "thickness", "on", "under", "front", "back", "left", "right", "vertical ", "horizontal", "top", "bottom", "inner", "outer", "clockwise", "counterclockwise", "axial", "radial", "circumferential", etc. indicate orientations or positional relationships based on those shown in the accompanying drawings, and are intended only to facilitate the description of the present disclosure and to simplify the description, and are not intended to indicate or imply that the device or element referred to must have a particular orientation, be constructed and operated in a particular orientation, and therefore cannot be be construed as a limitation of the present disclosure.

Furthermore, the terms "first" and "second" are used for descriptive purposes only and are not to be understood as indicating or implying order or relative importance, or implicitly specifying the number of technical features indicated. Thus, a feature defined with "first", "second" may expressly or impliedly include at least one such feature. In the description of the present disclosure, "multiple" means at least two, e.g., two, three, etc., unless otherwise expressly and specifically limited.

In this disclosure, unless otherwise expressly provided and limited, the terms "mount", "communicate", "connect", "fix", and the like are to be broadly construed. For example, it may be a fixed connection, a detachable connection, or integrating as a whole; it may be a mechanical connection, an electrical connection, or a communication connection; it may be a direct connection, an indirect connection through an intermediary medium, a connection within two elements, or an interaction between two elements, unless otherwise expressly limited. For those of ordinary skill in the art, the specific meaning of the above terms in the present disclosure may be understood on a case-by-case basis.

In the present disclosure, unless otherwise expressly provided and limited, the first feature being "on" or "under" the second feature may be a direct contact between the first and second features, or an indirect contact between the first and second features through an intermediate medium. Furthermore, the first feature being "on", "above" and "over" the second feature may be that the first feature is directly above or inclinedly above the second feature, or simply that the first feature is horizontally higher than the second feature. The first feature is "under", "below" and "underneath" the second feature may be that the first feature is directly below or inclinedly below the second feature, or simply that the first feature is horizontally lower than the second feature.

It should be noted that when an element is said to be "fixed to" or "disposed on" another element, it may be directly on the other element or there may be an intermediate element. When an element is said to be "connected" to another element, it may be directly connected to the other element or there may be an intermediate element. As used herein, the terms "vertical", "horizontal", "top", "bottom", "left," "right," and similar expressions are used herein for illustrative purposes only and are not intended to be the exclusive means of implementation.

An endoscope system having a fluorescence module is provided in an embodiment of the present disclosure. The endoscope system includes: an incident light module, an objective lens module, a relay lens module, a fluorescence module, a rear-end magnifying lens module, and a lens sheath coaxial module. The incident light module is fixedly connected to the fluorescence module, and provides perpendicular incident light to the fluorescence module. Along a direction perpendicular to the incident light, the rear-end magnifying lens module, the fluorescence module and the lens sheath coaxial module are sequentially arranged from back to front. The relay lens module and the objective lens module are sequentially arranged from back to front inside the lens sheath coaxial module. The fluorescence module includes: a cavity body fixedly connected to each of the rear-end magnifying lens module, the incident light module and the lens sheath coaxial module, and a dichroscope which is arranged, inside the cavity body, at an angle of 45 degrees with respect to a direction of the incident light. The incident light is totally reflected by the dichroscope, then passes through the relay lens module and reaches the objective lens module, and irradiates the surface of the object under observation, and the incident light is excited by a staining agent on the surface of the object under observation to generate reflected excited fluorescence; the excited fluorescence propagates through the objective lens module and the relay lens module and then reaches a surface of the dichroscope, and is fully transmitted through the dichroscope and enters the rear-end magnifying lens module.

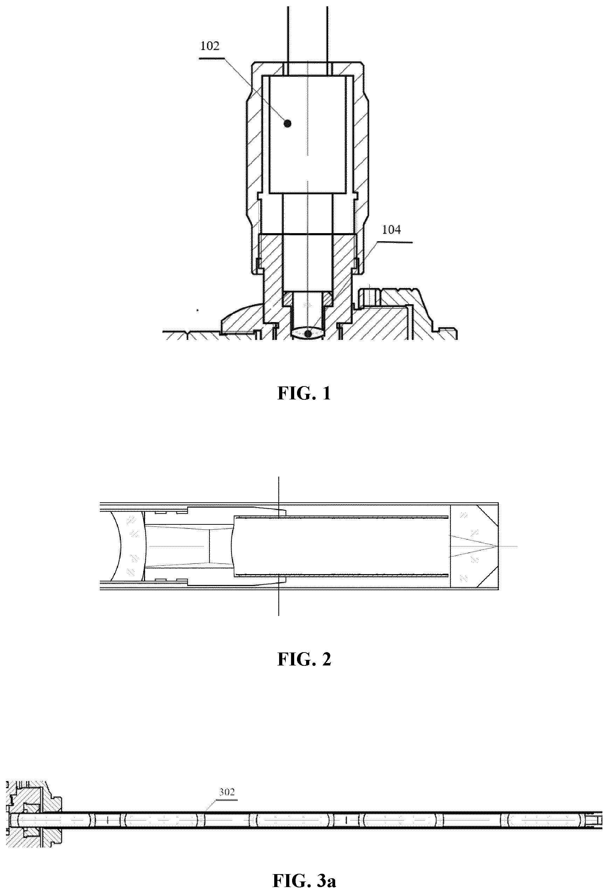

Specifically, as shown in FIG. 1, the incident light module includes an incident optical fiber 102 and an illumination lens 104. Light emitted through the incident optical fiber 102 passes through the illumination lens 104 and enters an excitation filter in the fluorescence module. The incident light module includes the incident optical fiber and the illumination lens. The incident light is emitted through the incident optical fiber, gets collected by the illumination lens, and then enters the fluorescence module. From an exit end face of the incident optical fiber, the incident light is imaged to a system diaphragm of an entire microendoscope through a combined optical system of the illumination lens and a part of the relay lens module; that is to say, there exists an object-image relationship between the exit end face of the incident optical fiber and the system diaphragm of the entire microendoscope, so as to facilitate the realization of Koehler illumination. The Koehler illumination enables uniform and sufficiently bright illumination onto the living biological tissue to be examined, and does not cause dazzling glare.

Specifically, in order to ensure that the image of the objective lens module can be transmitted over a long distance, the objective lens module adopts an image-side telecentric microscope objective system with a certain magnification. The higher the telecentricity of the image-side telecentric microscope objective system, the more helpful for imaging.

Specifically, as shown in FIG. 2, the objective lens module includes a small objective lens. The object surface generates fluorescence after being excited by an excitation light, and the fluorescence information emitted by the living biological tissue is imaged once by the small objective lens, and then imaged several times by the relay lens module, and enters the rear-end magnifying lens module. The excitation light emitted by the relay lens module passes through the objective lens module and uniformly illuminates the object surface, and at this time, light rays are not necessarily parallel; the fluorescence of the living biological tissue is excited, and the fluorescence information of the living biological tissue is collected by the objective lens module and imaged onto the image plane of the objective lens module, and then transmitted over a long distance by the relay lens module to the rear-end magnifying lens module. In order to ensure that the image of the objective lens can be transmitted over a long distance, the objective lens module needs to select an image-side telecentric system, and the higher the telecentricity, the more conducive to imaging.

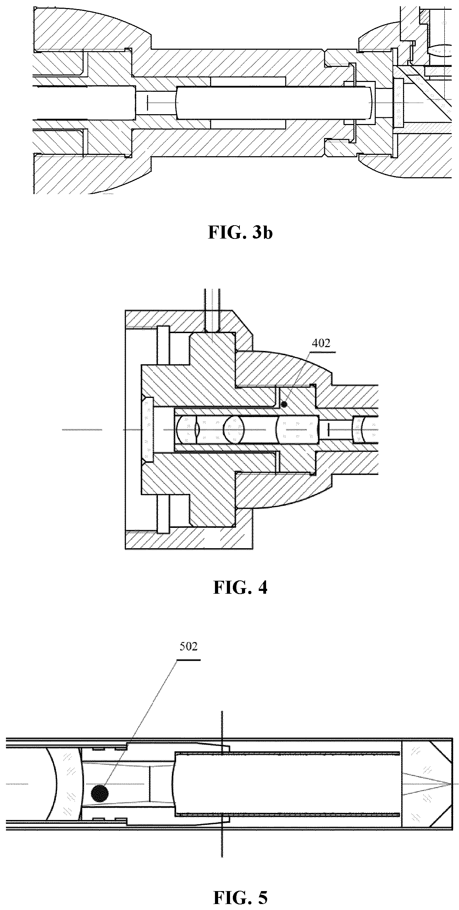

Specifically, the relay lens module includes a relay lens, the relay lens includes a rod lens set, each rod lens set includes two groups of rod lenses, and the two groups of rod lenses form a dual-telecentric imaging system with a target magnification. Furthermore, as shown in FIG. 3a and FIG. 3b, FIG. 3a shows a front end of the relay lens module, and FIG. 3b shows a rear end of the relay lens module, where 302 donates the rod lens, i.e., glued triplet lens, and the two groups of rod lenses form a dual-telecentric-1x imaging system, i.e., the magnification is 1. Multiple dual-telecentric imaging systems combine to form the relay lens, i.e., the relay lens includes even number of groups of rod lenses. An optical path among the rod lenses forming the dual-telecentric imaging system is quasi-parallel, i.e., not necessarily an absolutely parallel optical path. The overall magnification of the relay lens is 1X or -1X. Because the rod lenses are relatively small and lightweight, they can be easily held and manipulated, and the observer can use them more flexibly; the rod lenses can also provide a larger field of view, and observation can be performed in a narrow or difficult-to-reach space. The use of multiple groups of rod lenses to form the relay lens can realize the long-distance transmission of images, so that the object can be observed at a long distance, reducing the risk of close observation and avoiding the interference of the living organism to be examined.

Specifically, one particular dual-telecentric imaging system is selected from the relay lens, and the fluorescence module is placed in the parallel optical path of the selected dual-telecentric imaging system. Since the effect of the placement of the fluorescence module on the original relay lens imaging optical path is very small and almost negligible, placing the fluorescence module between the rod lenses that form a target dual-telecentric imaging system, while realizing the functions of generating the excitation light and filtering the fluorescence, etc., does not affect the original optical path, thereby reducing adverse effect on the image quality.

Specifically, the rear-end magnifying lens module includes a magnifying lens. As shown in FIG. 4, the rear-end magnifying lens module includes a combination 402 of a magnifying lens and a rod lens, and an object plane of the magnifying lens coincides with an image plane of the relay lens.

Specifically, the rear-end magnifying lens module further includes a camera device, the fluorescence being imaged on the camera device. Further, the camera device includes a complementary metal oxide semiconductor camera. Specifically, the camera device includes a Complementary Metal Oxide Semiconductor (CMOS) camera. Imaging using the CMOS camera has many advantages, not only in terms of production cost, but also CMOS has a shorter manufacturing cycle and better scalability. At the same time, CMOS can achieve better low-light and high-light control, with higher superiority and higher stability in high-resolution small devices, so it is suitable for endoscope systems.

Specifically, an object-side size of the magnifying lens corresponds to an image-side size of the camera device. Further, another function of the magnifying lens is to modulate an angle of main light rays emergent from the relay lens, so as to match an angle of main light rays of the CMOS, and to enhance fluorescence collection energy. The magnifying lens has a certain magnification so that the object-side size corresponds to the CMOS image-side size, enabling maximum utilization of the pixels.

Specifically, the endoscope system further includes an image processing module for processing an image formed on the camera device and outputting processed image data. Fluorescence entering the magnifying lens is directly imaged on the CMOS camera, and through the image processing module, an enlarged and clear image picture is output to improve the quality of the image picture.

Specifically, the lens sheath coaxial module includes a lens sheath and a coaxial clamping groove module. As shown in FIG. 5, the lens sheath coaxial module includes a lens sheath 502 and a coaxial clamping groove module (not shown in FIG. 5). Positions of respective lenses are effectively fixed by a coaxial clamping groove system at a front end inside the lens sheath through clamping. At the same time, the rod lens set and the objective lens set are held at a solid distance from each other by the coaxial clamping groove module, without displacement, and are overall on an identical circular axis. The lens sheath coaxial module is used to effectively fix the positions of respective lenses in the endoscope system through clamping, which can more firmly maintain the various components of the endoscope system, avoiding failures such as slipping off of the constituent lenses which may affect the performance of the endoscope.

Further, the lens sheath coaxial module also includes a detachable protective lens sheath, and the protective lens sheath is used to ensure the stability of the overall lens sheath. Specifically, on the basis of the internal lens sheath, a layer of protective lens sheath is sleeved on the outside of the internal lens sheath, where the protective lens sheath is detachable. The overall stability of the lens sheath is ensured by setting the protective lens sheath, and since the protective lens sheath is detachable, it is selectable to install or detach the protective lens sheath according to needs of different scenarios when using the endoscope system, improving the flexibility of the endoscope system.

In the embodiment, the endoscope system realizes medical examination of a living organism to be examined by means of an incident light module, an objective lens module, a relay lens module, a fluorescence module, and a rear-end magnifying lens module, and obtains clear and accurate examination images more efficiently. The fluorescence module includes a cavity body that is fixedly connected to each of the rear-end magnifying lens module, the incident light module and the lens sheath coaxial module, and a dichroscope disposed inside the cavity body at an angle of 45 degrees with respect to the direction of the incident light. The light path is that: the incident light enters the fluorescence module from the incident light module, the incident light is totally reflected by the dichroscope, then passes through the relay lens module and reaches the objective lens module, and irradiates the surface of the object under observation; the object surface of the objective lens module is excited by an excitation light to generate fluorescence, the fluorescence propagates through the objective lens module and the relay lens module and then reaches a surface of the dichroscope, and is fully transmitted through the dichroscope and enters the rear-end magnifying lens module. With the fluorescence module, incident light and emergent light propagate in an identical tubular cavity, and at the same time filtration of a specific fluorescence wave band is realized. In addition, modules in the endoscope system are fixed by the lens sheath coaxial module, ensure the stability of the endoscope system, avoiding problems such as falling off and disintegration in the process of use, and reducing the interference with the optoelectronic conversion of the rear-end magnifying lens and the interference caused by unstable installation of the lenses, so that a clearer image can be generated.

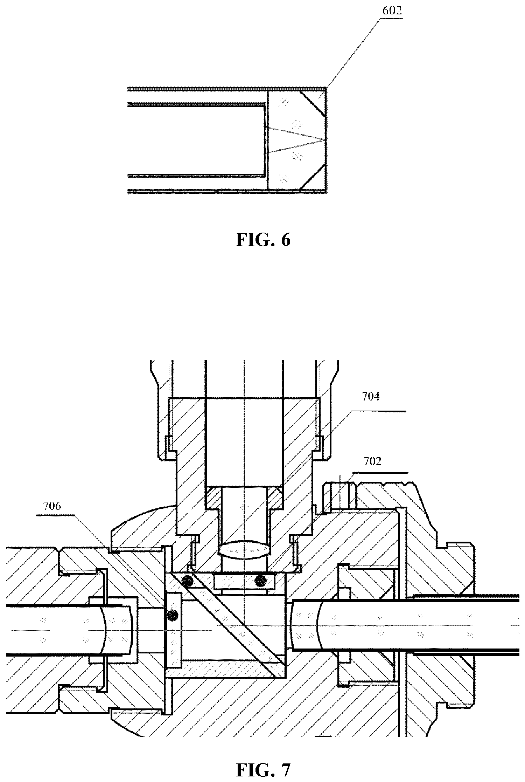

In an embodiment of the present disclosure, as shown in FIG. 6, a layer of protective lens 602 may be provided between the front end of the objective lens module and the object under observation, as an anti-shaking structure for preventing the endoscope system from experiencing a shaking phenomenon during use. The thickness of the protective lens depends on a working distance of the small objective lens in the objective lens module, and a thickest thickness cannot exceed the working distance of the small objective lens, and a thinnest thickness cannot be lower than 1.5 mm, to avoid the protective lens being exposed to the risk of rupture, i.e., 1.5 mm ≤ the thickness of the anti-shaking structure ≤ the working distance of the small objective lens in the objective lens module.

Specifically, glass is selected as a material for the protective lens, and other light-transmitting homogeneous medium materials may also be used instead.

In the embodiment, the protective lens is designed at the front end of the objective lens module, and the design point of the width of the protective lens is to ensure that the incident light will converge on the surface of the object under observation after the incident light is emitted from the objective lens module, and that the negative effects caused by shaking can be effectively prevented by pressing the protective lens against the surface of the living organism under observation. In order to be able to add an anti-shaking lens sheath structure to the objective lens module, the objective lens module will first be set up as an objective lens system with negative spherical aberration, and the spherical aberration will be compensated for by a piece of dielectric lens at a front end of the anti-shaking structure; on the other hand, a terminal of the lens sheath extends beyond the protective lens, and when attached to the surface of the living organism, a periphery of the terminal of the lens sheath abuts against a periphery of the observation area, which further prevents the negative effects of shaking.

In an embodiment of the present disclosure, the fluorescence module also includes an excitation filter set above the dichroscope and a fluorescence filter set at a back end of the dichroscope. Incident light strikes vertically onto the excitation filter to generate incident light of 470 nm~480 nm, and the fluorescence filter performs a secondary filtering on excited fluorescence after passing through the dichroscope and generates excited fluorescence of 525 nm±20 nm.

Specifically, as shown in FIG. 7, the fluorescence module includes an excitation filter 702, a dichroscope 704, and a fluorescence filter 706, where the fluorescence filter may be replaced by an emission filter. The incident light enters the fluorescence module in the endoscope system through the incident light module, an excitation light is obtained through the excitation filter 702 in the fluorescence module, the dichroscope 704 reflects the excitation light into the relay lens module, and then the excitation light enters the objective lens module from the relay lens module and uniformly illuminates a position of the object surface. The excitation light excites a fluorescence of the living biological tissue, and fluorescence information of the living biological tissue is collected by the objective lens and imaged onto an image plane of the objective lens; the fluorescence is further transmitted over a long distance by the relay lens module, filtered by the fluorescence filter 706 after passing through the dichroscope 704, and transmitted to the rear-end magnifying lens module.

In the embodiment, the excitation filter is used to generate excitation light to maximize the excitation efficiency of an excited substance. The fluorescence band after excitation by staining agent on the surface of the organism is in the vicinity of 525 nm, but the dichroscope cannot achieve 100% filtering of the light, so the fluorescence filter is added after the dichroscope for further filtering of the light in order to improve the imaging effect.

In an embodiment of the present disclosure, the cavity body includes a first connection cavity that opens upwardly for fixedly connecting the incident light module, a second connection cavity that opens backwardly for connecting the rear-end magnifying lens module, and a third connection cavity that opens forwardly for connecting the lens sheath coaxial module; and the first connection cavity, the second connection cavity, and the third connection cavity converge in the interior of the cavity body and form an accommodating cavity.

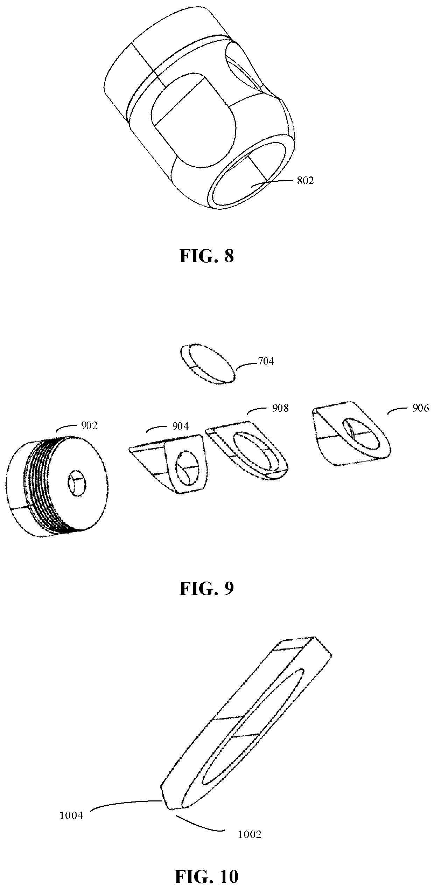

Specifically, as shown in FIG. 8, the accommodating cavity 802 is used to accommodate the dichroscope and a fixing structure assembly for the dichroscope.

In the embodiment, three-way openings of an intermediate cavity, i.e. the first connection cavity, the second connection cavity and the third connection cavity are used for fixing and connecting relevant modules of the endoscope, respectively, and the accommodating cavity facilitates the installation of the dichroscope and the fixing structure assembly for the dichroscope.

In an embodiment of the present disclosure, the fluorescence module further includes a first fixing member disposed at the front end of the dichroscope and a second fixing member disposed at the back end of the dichroscope, the first fixing member and the second fixing member are affixed to the dichroscope in opposite directions, and the first and second fixing members and the dichroscope are accommodated in the accommodating cavity as a whole.

Exemplarily, as shown in FIG. 9, the dichroscope adopts a self-impacted structure. The self-impacted structure includes the dichroscope 704, a fixing connector 902, an upper-left half-triangular fixing member 904, and a lower-right half-triangular fixing member 906. The upper-left half-triangular fixing member 904 is the first fixing member disposed at the front end of the dichroscope, and the lower-right half-triangular fixing member 906 is the second fixing member disposed at the back end of the dichroscope. Two half-triangular tapered fixing members, which hold the dichroscope 704 therebetween, combine to form the fixing structure assembly for the dichroscope 704 located therein, where the fixing structure assembly is accommodated in the intermediate accommodating cavity. A left side of the upper-left half-triangular fixing member 904 abuts against a left side of an inner wall of the accommodating cavity, and a right side of the inner wall of the accommodating cavity impacts, through the fixing connector 906, an entire assembly of dichroscope within the accommodating cavity. The fixing connector 902 may be fixedly connected to the accommodating cavity by means of, for example, screwing, riveting or the like.

In the embodiment, the first fixing member and the second fixing member are affixed to the dichroscope in opposite directions to form the self-impacted structure, ensuring that the dichroscope can be fixed, and reducing deviation of fluorescence light path which is caused by displacement of the dichroscope and may affect the imaging quality of the endoscope.

In an embodiment of the present disclosure, the fluorescence module also includes a fixing washer disposed around a periphery of the dichroscope. The dichroscope is housed in the fixing washer, and the fixing washer and the dichroscope are clamped between the first fixing member and the second fixing member as a whole, forming a fixing module.

Specifically, as shown in FIG. 9, the self-impacted structure also includes a metallic fixing washer 908. The upper-left half-triangular fixing member 904 and the lower-right half-triangular fixing member 906 that hold the dichroscope 704 therebetween, as well as the metallic fixing washer 908 combine to form the fixing structure assembly for the dichroscope located therein, where the fixing structure assembly is accommodated in the intermediate accommodating cavity. The fixing washer includes a vertical limiting face and a circularly curved fitting face. The fixing washer together with the first fixing member and the second fixing member, and the dichroscope form a three-clamp-one structure.

In the embodiment, the dichroscope is housed in the fixing washer, and the fixing washer and the dichroscope are clamped between the first fixing member and the second fixing member as a whole, forming a fixing module; hence, the dichroscope is protected and further fixed at the same time.

In an embodiment of the present disclosure, the fixing module includes a locating face matching the inner wall of the accommodating cavity. The locating face includes a horizontally disposed rectangular upper locating face and a horizontally disposed lower locating face having a semi-circular or semi-elliptical cross-section. The fixing washer includes a horizontally disposed upper support portion which forms a part of the upper locating face, and a horizontally disposed lower support portion which forms a part of the lower locating face.

In the embodiment, a position of the fixing washer is determined based on the locating faces matching the inner wall of the accommodating cavity to ensure the accuracy of the position of the fixing washer.

In an embodiment of the present disclosure, a bottom end of the lower support portion is provided with a first limiting face that fits with a lower inner surface of the cavity body and partially forms the lower locating face, and a second limiting face that perpendicularly intersects with the first limiting face and fits with a front inner surface of the cavity body.

Specifically, as shown in FIG. 10, the lower support portion of the fixing washer includes a first limiting face 1002 and a second limiting face 1004.

In the embodiment, the first limiting face and the second limiting face further ensure that the self-impacted structure for the dichroscope has effects of fixing and stable clamping, so that the dichroscope is not displaced. Here, the limiting faces, also referred as limiting cutoff faces, are fixedly connected to respective modules and limit positions of the respective modules.

In an embodiment of the present disclosure, a first mounting hole is formed in the first fixing member from top to bottom, and a first cutoff face is formed above the dichroscope, and the excitation filter is mounted within the first mounting hole and cuts off at the first cutoff face.

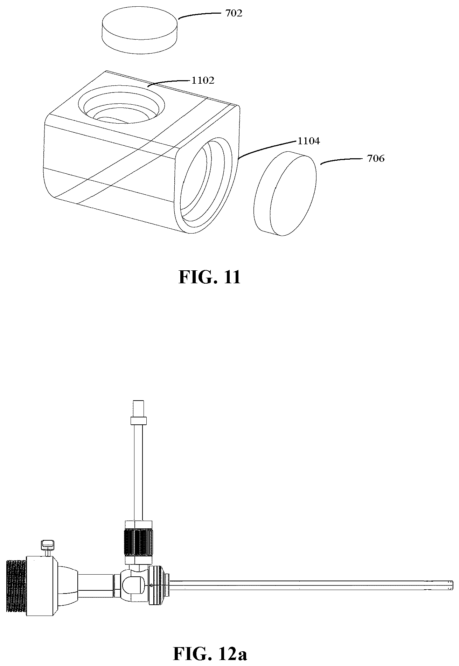

Specifically, as shown in FIG. 11, a first mounting hole 1102 is formed in the first fixing member from top to bottom. Values about the first mounting hole 1102 such as size, radius, depth correspond to the laser filter.

In the embodiment, the installation of the laser filter is achieved by forming the mounting hole with values corresponding to the laser filter, which can ensure that the laser filter can not move, so the image quality may not be affected.

In an embodiment of the present disclosure, a second mounting hole is formed in the second fixing member from back to front, and a second cutoff face is formed at the rear of the dichroscope. The fluorescence filter is mounted within the second mounting hole and cuts off at the second cutoff face.

Specifically, still as shown in FIG. 11, the second mounting hole 1104 is formed in the second fixing member from back to front. Values about the second mounting hole 1104 such as size, radius, depth correspond to the fluorescence filter.

In the embodiment, the installation of the fluorescence filter is achieved by forming the mounting hole with values corresponding to the fluorescence filter, thereby ensuring that the fluorescence filter can not move and the image quality may not be affected.

In an embodiment of the present disclosure, a blocking face is formed at a position where each of the first connection cavity, the second connection cavity and the third connection cavity connects to the cavity body; the incident light module is fixedly connected to the cavity body through the first connection cavity and cuts off at a corresponding blocking face, the rear-end magnifying lens module is fixedly connected to the cavity body through the second connection cavity and cuts off at a corresponding blocking face, and the lens sheath coaxial module is fixedly connected to the cavity body through the third connection cavity and cuts off at a corresponding blocking face.

In the embodiment, the fixation effect of components of the endoscope system is further strengthened according to spatial relationships between the incident light module, the rear-end magnifying lens module and the lens sheath coaxial module, and the blocking faces respectively corresponding to the first connection cavity, the second connection cavity and the third connection cavity.

In an embodiment of the present disclosure, there is provided an endoscope system having a fluorescence module. The endoscope system includes an incident light module, an objective lens module, a relay lens module, the fluorescence module, a rear-end magnifying lens module, and a lens sheath coaxial module. The incident light module is fixedly connected to the fluorescence module and provides perpendicular incident light to the fluorescence module. Along a direction perpendicular to the incident light, the rear-end magnifying lens module, the fluorescence module and the lens sheath coaxial module are sequentially arranged from back to front; the relay lens module and the objective lens module are sequentially arranged from back to front inside the lens sheath coaxial module. The fluorescence module includes a cavity body fixedly connected to each of the rear-end magnifying lens module, the incident light module and the lens sheath coaxial module, and a dichroscope which is arranged, inside the cavity body, at an angle of 45 degrees with respect to a direction of the incident light. The incident light is totally reflected by the dichroscope, then passes through the relay lens module and reaches the objective lens module, and irradiates a surface of an object under observation, and the incident light is excited by a staining agent on the surface of the object under observation to generate reflected excited fluorescence. The excited fluorescence propagates through the objective lens module and the relay lens module and then reaches a surface of the dichroscope, and is fully transmitted through the dichroscope and enters the rear-end magnifying lens module. Specifically, the incident light module includes an incident optical fiber and an illumination lens, and the incident light enters the fluorescence module through the incident optical fiber and the illumination lens. The fluorescence module also includes an excitation filter set above the dichroscope and a fluorescence filter set at a back end of the dichroscope, and the incident light strikes vertically onto the excitation filter to generate incident light of 470 nm~480 nm, and the fluorescence filter performs a secondary filtering on excited fluorescence after passing through the dichroscope and generates excited fluorescence of 525 nm±20 nm. The cavity body includes an opening upward to securely connect the incident light module to a first cavity body. The cavity body includes a first connection cavity that opens upwardly for fixedly connecting the incident light module, a second connection cavity that opens backwardly for connecting the rear-end magnifying lens module, and a third connection cavity that opens forwardly for connecting the lens sheath coaxial module, and the first connection cavity, the second connection cavity, and the third connection cavity converge in interior of the cavity body and form an accommodating cavity. The fluorescence module also includes a first fixing member disposed at a front end of the dichroscope and a second fixing member disposed at a back end of the dichroscope, the first fixing member and the second fixing member are affixed to the dichroscope in opposite directions, and the first and second fixing members and the dichroscope are accommodated in the accommodating cavity as a whole. The fluorescence module also includes a fixing washer disposed around a periphery of the dichroscope, the dichroscope is housed in the fixing washer, and the fixing washer and the dichroscope are clamped between the first fixing member and the second fixing member as a whole, forming a fixing module. The fixing module includes a locating face matching an inner wall of the accommodating cavity, the locating face includes a horizontally disposed upper locating face which is rectangular and a horizontally disposed lower locating face having a semi-circular or semi-elliptical cross-section, and the fixing washer includes a horizontally disposed upper support portion which forms a part of the upper locating face, and a horizontally disposed lower support portion which forms a part of the lower locating face. A bottom end of the lower support portion is provided with a first limiting face that fits with a lower inner surface of the cavity body and partially forms the lower locating face, and a second limiting face that perpendicularly intersects with the first limiting face and fits with a front inner surface of the cavity body. A first mounting hole is formed in the first fixing member from top to bottom, and a first cutoff face is formed above the dichroscope; the excitation filter is mounted within the first mounting hole and cuts off at the first cutoff face. A second mounting hole is formed in the second fixing member from back to front, and a second cutoff face is formed at the rear of the dichroscope; the fluorescence filter is mounted within the second mounting hole and cuts off at the second cutoff face. A blocking face is formed at a position where each of the first connection cavity, the second connection cavity and the third connection cavity connects to the cavity body. The incident light module is fixedly connected to the cavity body through the first connection cavity and cuts off at a corresponding blocking face, the rear-end magnifying lens module is fixedly connected to the cavity body through the second connection cavity and cuts off at a corresponding blocking face, and the lens sheath coaxial module is fixedly connected to the cavity body through the third connection cavity and cuts off at a corresponding blocking face.

Further, the incident light is excited by the excitation filter to obtain the excitation light, the excitation light is reflected by the dichroscope to the relay lens module and the objective lens module, and irradiates the object surface of the objective lens module. The objective lens module adopts an image-side telecentric microscope objective system, and the objective lens module includes a small objective lens. The object surface of the objective lens module produces the fluorescence after being excited by the excitation light. The fluorescence is imaged once by the small objective lens, and then imaged by the relay lens module in accordance with a target number of times, and transmits through the excited and is filtered by the fluorescence filter, and then enters the rear-end magnifying lens module. Specifically, the relay lens module includes a relay lens, the relay lens includes a rod lens set, each rod lens set includes two groups of rod lenses, and the two groups of rod lenses form a dual-telecentric imaging system with a target magnification. A target dual-telecentric imaging system is selected, with the fluorescence module disposed between the rod lenses forming the target dual-telecentric imaging system. Specifically, the rear-end magnifying lens module includes a magnifying lens, an object plane of the magnifying lens coinciding with an image plane of the relay lens. The rear-end magnifying lens module further includes a camera device, the fluorescence imaging being on the camera device. The camera device includes a CMOS camera, an object-side size of the magnifying lens corresponding to an image-side size of the camera device. Specifically, the lens sheath coaxial module includes a lens sheath, and a coaxial clamping groove module for fixing each module in the endoscope system. The lens sheath coaxial module also includes a detachable protective lens sheath, and the protective lens sheath is used to ensure the stability of the overall lens sheath. Further, the endoscope system further includes an image processing module for processing images formed on the camera device and outputting the processed image data.

In some embodiments, the incident light enters the endoscope system through the incident optical fiber, and enters the fluorescence module through the illumination lens. The incident light exits the illumination lens, is filtered by the excitation filter, then reflected by the dichroscope into the relay lens, and then enters the small objective lens and uniformly illuminates the position of the object surface. The object surface is excited by the excitation light to produce fluorescence, the fluorescence information emitted by the living biological tissue is imaged once by the small objective lens, and then is imaged multiple times by the relay lens and enters the magnifying lens. The dichroscope and the fluorescence filter are located between the relay lenses, the fluorescence transmits through the dichroscope and gets filtered by the fluorescence filter filter, the fluorescence entering the magnifying lens is directly imaged in the CMOS camera, then through the image processing system, a magnified clear image picture is output.

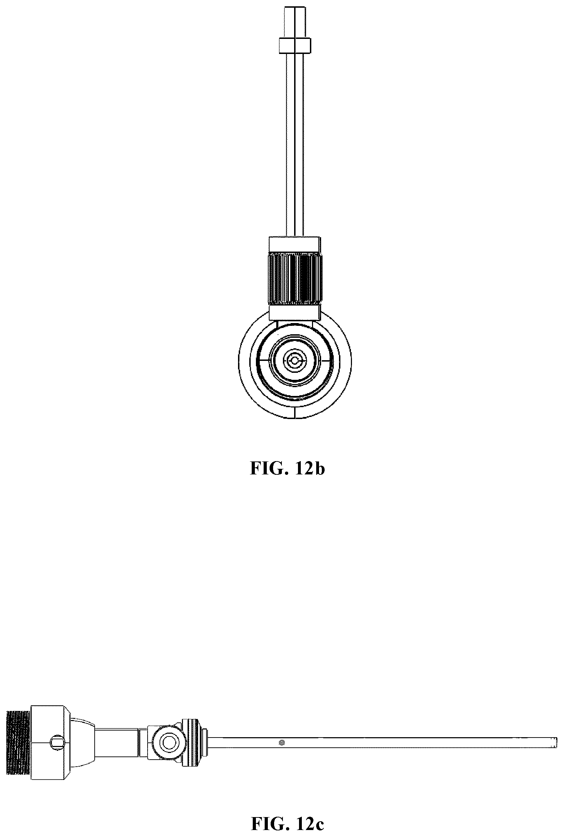

In some embodiments, as shown in FIGS. 12a, 12b, and 12c, three-view drawings of an endoscope system having a fluorescence module are provided. FIG. 12a shows a front view of the endoscope system having the fluorescence module, FIG. 12b shows a right view of the endoscope system having the fluorescence module, and FIG. 12c shows an upper view of the endoscope system having the fluorescence module.

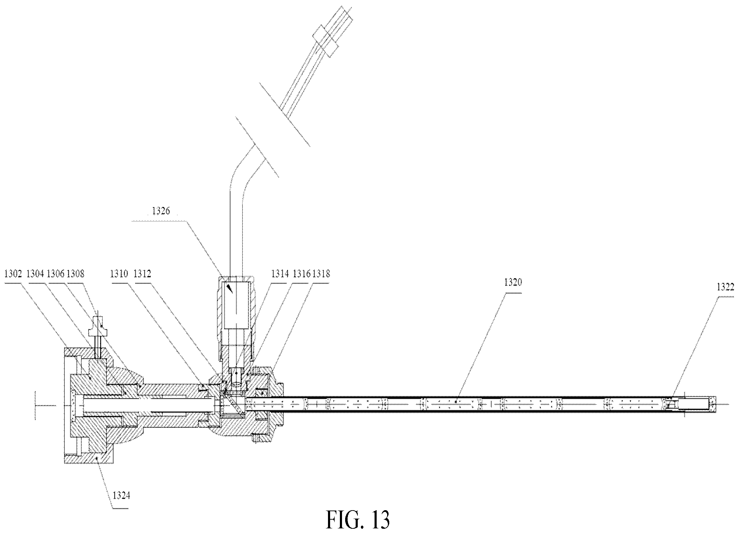

Further, as shown in FIG. 13, a sectional structure of an endoscope system having a fluorescence module includes a camera holder component 1302, a 4x magnifying lens and rod lens set 1304, a connecting tube 1306, a pin 1308, a compression ring 1310, a lens body 1312, a fluorescence module 1314, a cold light source coaxial illuminator 1316, a connecting ring 1318, a rod lens component and a 3X0.45 small objective lens 1320, lens sheath 1322, camera pressure cap 1324 and illumination optical fiber 1326, where the illumination optical fiber is the incident optical fiber.

The above-described embodiments and the various technical features in the above-described embodiments may be combined arbitrarily, and for the sake of conciseness of description, not all possible combinations of the various technical features in the above-described embodiments have been described, however, as long as there is no contradiction in the combinations of these technical features, they should all be considered to be within the scope of the present specification as recorded herein.

The above-described embodiments express only several embodiments of the present application, which are described in a more specific and detailed manner, but are not to be construed as a limitation of the scope of the patent application. It should be pointed out that, for a person of ordinary skill in the art, several deformations and improvements can be made without departing from the conception of the present application, all of which fall within the scope of protection of the present application. Therefore, the scope of protection of the patent application shall be subject to the appended claims.

## Claims

1. An endoscope system having a fluorescence module, **characterized by** comprising: an incident light module, an objective lens module, a relay lens module, the fluorescence module, a rear-end magnifying lens module, and a lens sheath coaxial module; wherein the incident light module is fixedly connected to the fluorescence module and provides perpendicular incident light to the fluorescence module; along a direction perpendicular to the incident light, the rear-end magnifying lens module, the fluorescence module and the lens sheath coaxial module are sequentially arranged from back to front; the relay lens module and the objective lens module are sequentially arranged from back to front inside the lens sheath coaxial module; wherein the fluorescence module comprises a cavity body fixedly connected to each of the rear-end magnifying lens module, the incident light module and the lens sheath coaxial module, and a dichroscope which is arranged, inside the cavity body, at an angle of 45 degrees with respect to a direction of the incident light; wherein the incident light is totally reflected by the dichroscope, then passes through the relay lens module and reaches the objective lens module, and irradiates a surface of an object under observation, and the incident light is excited by a staining agent on the surface of the object under observation to generate reflected excited fluorescence; and wherein the excited fluorescence propagates through the objective lens module and the relay lens module and then reaches a surface of the dichroscope, and is fully transmitted through the dichroscope and enters the rear-end magnifying lens module.

2. The endoscope system according to claim **1,** wherein the fluorescence module further comprises an excitation filter set above the dichroscope and a fluorescence filter set at a back end of the dichroscope, wherein the incident light strikes vertically onto the excitation filter to generate incident light of 470 nm~480 nm, and the fluorescence filter performs a secondary filtering on excited fluorescence after passing through the dichroscope and generates excited fluorescence of 525 nm±20 nm.

3. The endoscope system according to claim 2, wherein the cavity body comprises a first connection cavity that opens upwardly for fixedly connecting the incident light module, a second connection cavity that opens backwardly for connecting the rear-end magnifying lens module, and a third connection cavity that opens forwardly for connecting the lens sheath coaxial module, wherein the first connection cavity, the second connection cavity, and the third connection cavity converge in interior of the cavity body and form an accommodating cavity.

4. The endoscope system according to claim 3, wherein the fluorescence module further comprises a first fixing member disposed at a front end of the dichroscope and a second fixing member disposed at a back end of the dichroscope, the first fixing member and the second fixing member are affixed to the dichroscope in opposite directions, and the first and second fixing members and the dichroscope are accommodated in the accommodating cavity as a whole.

5. The endoscope system according to claim 4, wherein the fluorescence module further comprises a fixing washer disposed around a periphery of the dichroscope, the dichroscope is housed in the fixing washer, and the fixing washer and the dichroscope are clamped between the first fixing member and the second fixing member as a whole, forming a fixing module.

6. The endoscope system according to claim 5, wherein the fixing module comprises a locating face matching an inner wall of the accommodating cavity, the locating face comprises a horizontally disposed upper locating face which is rectangular and a horizontally disposed lower locating face having a semi-circular or semi-elliptical cross-section, and the fixing washer comprises a horizontally disposed upper support portion which forms a part of the upper locating face, and a horizontally disposed lower support portion which forms a part of the lower locating face.

7. The endoscope system according to claim 6, wherein a bottom end of the lower support portion is provided with a first limiting face that fits with a lower inner surface of the cavity body and partially forms the lower locating face, and a second limiting face that perpendicularly intersects with the first limiting face and fits with a front inner surface of the cavity body.

8. The endoscope system according to claim 4, wherein a first mounting hole is formed in the first fixing member from top to bottom, and a first cutoff face is formed above the dichroscope, and wherein the excitation filter is mounted within the first mounting hole and cuts off at the first cutoff face.

9. The endoscope system according to claim 4, wherein a second mounting hole is formed in the second fixing member from back to front, and a second cutoff face is formed at the rear of the dichroscope, and wherein the fluorescence filter is mounted within the second mounting hole and cuts off at the second cutoff face.

10. The endoscope system according to claim 8 or claim 9, wherein a blocking face is formed at a position where each of the first connection cavity, the second connection cavity and the third connection cavity connects to the cavity body, wherein the incident light module is fixedly connected to the cavity body through the first connection cavity and cuts off at a corresponding blocking face, the rear-end magnifying lens module is fixedly connected to the cavity body through the second connection cavity and cuts off at a corresponding blocking face, and the lens sheath coaxial module is fixedly connected to the cavity body through the third connection cavity and cuts off at a corresponding blocking face.
